(19) Europäisches Patentamt
European Patent Office
Office européen des brevets

(11) EP 2 083 796 B1

(12) EUROPEAN PATENT SPECIFICATION

(45) Date of publication and mention
of the grant of the patent:
**16.03.2011 Bulletin 2011/11**

(51) Int Cl.:
*A61P 17/00* (2006.01)     *A61K 33/38* (2006.01)
*A61K 33/06* (2006.01)     *A61K 33/24* (2006.01)
*A61K 33/26* (2006.01)     *A61K 33/28* (2006.01)
*A61K 33/30* (2006.01)     *A61K 33/32* (2006.01)
*A61K 33/36* (2006.01)     *A61K 9/51* (2006.01)
*A61K 9/00* (2006.01)     *A61K 9/70* (2006.01)
*A61L 15/18* (2006.01)     *A61K 45/06* (2006.01)
*A61K 47/48* (2006.01)

(21) Application number: **07868587.2**

(22) Date of filing: **26.10.2007**

(86) International application number:
**PCT/US2007/082603**

(87) International publication number:
**WO 2008/055049 (08.05.2008 Gazette 2008/19)**

(54) **METAL COATED NANOPARTICLES FOR USE IN THE TREATMENT OF ENZYMATIC DERMATITIS**

METALLBESCHICHTETE NANOPARTIKELN ZUR ANWENDUNG IN DER BEHANDLUNG DER ENZYMATISCHEN DERMATITIS

NANOPARTICULES A COUCHE METALLIQUE POUR UTILISATION DANS LE TRAITEMENT DE LA DERMATITE ENZYMATIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.10.2006 US 554214**

(43) Date of publication of application:
**05.08.2009 Bulletin 2009/32**

(73) Proprietor: **McNeil-PPC, Inc.**
**Skillman, NJ 08558 (US)**

(72) Inventors:
• **DI SALVO, Anthony**
**Bernardsville, NJ 07924 (US)**
• **MORDAS, Carolyn J.**
**Lawerenceville, NJ 08648 (US)**
• **NIKOLOVSKI, Janeta**
**Princeton, NJ 08542 (US)**
• **WIEGAND, Benjamin C.**
**Yardley, PA 19067 (US)**

(74) Representative: **Kirsch, Susan Edith et al**
**Carpmaels & Ransford**
**One Southampton Row**
**London**
**WC1B 5HA (GB)**

(56) References cited:
**EP-A- 0 117 613     EP-A- 0 414 605**
**WO-A-01/52842     WO-A-2005/042041**
**US-A1- 2003 104 019     US-A1- 2005 115 462**
**US-A1- 2005 129 624     US-A1- 2005 175 649**

• **BUCKINGHAM K W ET AL: "ETIOLOGIC FACTORS IN DIAPER DERMATITIS: THE ROLE OF FECES" PEDIATRIC DERMATOLOGY, BLACKWELL SCIENTIFIC PUBLICATIONS, BOSTON, MA, US, vol. 3, no. 2, 1 February 1986 (1986-02-01), pages 107-112, XP000995228 ISSN: 0736-8046**

## Description

Field of the Invention

The invention relates to composite materials for treating skin conditions, such as enzymatic dermatitis, that typically arise from wearing absorbent articles such as diapers, training pants, adult incontinence briefs, feminine hygiene products and the like.

Background of the Invention

Diaper rash is a common form of irritation and inflammation of those parts of an infant's body normally covered by a diaper. This condition is also referred to as diaper dermatitis, napkin dermatitis, napkin rash or nappy rash. While certainly more common in infants, this condition is not, in fact, limited to infants. Any individual who suffers from incontinence to the extent that the use of absorbent articles is required may develop this condition. Susceptible individuals range from newborns, to the elderly, to critically ill or nonambulatory individuals. 21 C.F.R. 333.503 defines diaper rash as "[a]n inflammatory skin condition in the diaper area (perineum, buttocks, lower abdomen, and inner thighs) caused by one or more of the following factors: moisture, occlusion, chafing, continued contact with urine or feces or both, or mechanical or chemical irritation." It is generally accepted by the medical profession that true diaper rash or diaper dermatitis is a condition which is, in its most simple stages, a contact irritant dermatitis resulting from extended contact of the skin with urine, or feces, or both. Among the most commonly accepted factors linked to diaper rash are ammonia, fecal enzymes, bacteria, the products of bacterial action, urine pH, and c. *albicans.*

Because enzymes are widely distributed in plants, molds, bacteria, milk, milk products, and almost all animal tissues as well as in digestive juices in the gastrointestinal tract, they are almost always present in connection with diapers, incontinence articles, sanitary napkins, liners and so forth. Such enzymes include proteases, lipases and other esterases and diesterases, ureases and other enzymes including amylases, elastases, nucleases, and the like. Although the relative contribution of the different types of enzymes to skin irritation is unknown, there is evidence that at least fecal proteases and lipases of intestinal and/or pancreatic origin play a direct role in causing the skin inflammation of enzymatic dermatitis. Similarly, proteases are known to be present within mentral fluid. They result from breakdown of the uterine lining.

Studies with inhibitors designed to inhibit the enzymatic activity of various classes of proteases have shown that serine proteases, cysteine proteases and metalloproteases were the most likely to be responsible for the overall proteolytic activity of feces. MMPs, matrix metalloproteases, are also most likely to be present in menstral fluid. It is known that the serine proteases trypsin and chymotrypsin, in particular, are nearly always present in grossly measurable quantities in the stools of normal young children, and smaller but detectable quantities are present in normal adult stools. Lipases, including esterases that hydrolyze dietary triglycerides, are also found in normal stools and are capable of hydrolizing triglycerides and other glycerides found in human skin to form irritating fatty acid and glycerol by-products. Thus, when skin is exposed to enzymes such as lipases and proteases present in body exudates, lipid-containing components and protein-containing components of the skin, especially of the barrier layer (stratum corneum), can be broken down resulting in the irritation and inflammation of diaper rash. Moreover, perturbation of the skin barrier allows other components of urine and feces, ammonia, bacteria, yeast and the like which may not otherwise be irritating by themselves, to migrate through the compromised skin barrier to produce additional irritation and possible infection.

For centuries, silver metal has been known to be an agent capable of killing many different microbial species, including *c. albicans* and *s. aureus,* which are commonly found in skin areas affected by diaper rash. Silver metal has been used to purify drinking solutions or administered to sick individuals before the existence of modern antibiotics. Even after the discovery of penicillin and its descendents, colloidal silver solutions were often used in cases in which troublesome bacteria had become resistant to antibiotics.

Colloidal silver solutions are commercially available today, however they are often unstable, and have a short shelf life. This is due to the tendency of the silver particles to aggregate and form clusters so large that they are no longer suspended in solution. For this reason, undesirable gelling agents are added to solutions to keep the silver particles suspended by preventing particle aggregation. Another problem of the commercially available solutions is that the majority of the silver content is usually found to be silver ions. Though ionic silver is an effective anti-microbial agent, its presence in medical and personal care applications poses a problem because silver ions rapidly combine with ubiquitous chloride to form an insoluble white precipitate and can tint the skin grayish-blue.

Commercially available diaper rash compositions are based on petrolatum and/or zinc oxide.

US 2003/0104019 discloses swellable clay-containing compositions as effective in reducing the enzymatic irritation to the skin.

Nanoparticles are known to be used as fillers as disclosed in US Pat. No. 6,492,453, as coatings as disclosed in US 2003/0185964 and as foam components as disclosed in US Pat. No. 6,518,324.

Inorganic particulates, such as, clays, silicates, and alumina have been widely used in combination with adjunct detergent and laundry compounds to impart some form of antistatic control and/or fabric softening benefit. Similarly, nanoparticle systems are disclosed in US 2002/0150678 for imparting surface modifying benefits to soft and hard surfaces, for example laundry detergents.

US 2005/0129624 A discloses silver nanocrystalline materials against skin disorders, also in view of its MMP inhibiting properties. The material is not coated onto particles but as a solution, powder or gel or as such on dressings. There is no specific mention of enzymatic dermatitis (albeit contact irritant dermatitis is mentioned).

US 2005/0115462 discloses functionalized nanoparticles incorporated into soft surface coatings used in the preparation of improved absorbent articles, such as pantiliners, sanitary napkins, interlabial devices, adult incontinent devices, breast pads, shoe insoles, bandages, and diapers. The articles comprise a composite material formed from (a) an exfoliated nanoparticle having a surface and (b) a metal selected from Groups 3 to 12, aluminum and magnesium, where the metal is loaded onto the surface of the nanoparticle. The composite material may be applied to the absorbent article, for example a layer thereof, in the form of a soft surface coating.

It has now been surprisingly found that enzymatic dermatitis, for example diaper dermatitis caused by fecal enzymes, can be treated by topical application of such composite materials. In one embodiment, the composite material comprises silver loaded onto a nanoclay. Silver-coated nanoclays in particular have excellent enzyme inhibiting properties, especially when applied to the skin.

Summary of the invention

The invention provides a composition with an effective amount of a composite material comprising: (a) an exfoliated nanoparticle having a surface, and (b) a metal selected from Groups 3 to 12, aluminum and magnesium, wherein the metal is loaded onto the surface of the nanoparticle.

The invention also provides a composite material comprising: (a) an exfoliated nanoparticle having a surface and (b) a metal selected from Groups 3 to 12, aluminum and magnesium, wherein the metal is loaded onto the surface of the nanoparticle for use in treating enzymatic dermatitis, comprising topically applying to an area of skin affected with enzymatic dermatitis an effective amount of a composite material.

Detailed Description of the Invention

Every limit given throughout this specification includes every lower or higher limit, as the case may be, as if such lower or higher limit was expressly written herein. Every range given throughout this specification includes every narrower range that falls within such broader range, as if such narrower ranges were all expressly written herein.

As used herein, the phrase "skin conditions" are those resulting from enzymatic dermatitis such as diaper or skin rash, including the physical side effects of irritation, redness, itching, burning, or inflammation.

As used herein, the phrase "method of treating" shall include methods for preventing, reducing, or curing conditions. Such methods shall include any methods that reduce or lessen the skin conditions mentioned above.

As used herein, the phrase "absorbent articles" are those articles used by infants and adults to absorb body wastes, fluids and exudates. Included are diapers (both infant and adult), sanitary protection articles such as pantiliners and full sized absorbent napkins (including ultrathin types), and wound care articles such as surgical gauze and bandages. The absorbent articles may absorb fluid but also contain solid or semi-solid waste such as feces.

As used herein, the term "nanoparticles" are those particles (including but not limited to rod-shaped particles, disc-shaped particles, platelet-shaped particles, tetrahedral-shaped particles), fibers, nanotubes, or any other materials having dimensions on the nano scale.

The present invention describes a composite material for treating skin conditions arising from the skin barrier disruption caused by enzymes, in particular lipases and proteases. The present invention is effective in reducing the activity of such enzymes.

The composite material may be used in a method comprising contacting an enzyme selected from the group consisting of lipases and proteases with an effective amount of a composite material comprising: (a) an exfoliated nanoparticle having a surface, and (b) a metal selected from Groups 3 to 12, aluminum and magnesium, wherein the metal is loaded onto the surface of the nanoparticle.

In the present invention, the nanoparticles have an average particle size of 1 to 1000 nanometers, preferably 2 to 750 nanometers. That is, the nanoparticles have a largest dimension (e.g., a diameter or length) of 1 to 1000 nm. Nanotubes can include structures up to 1 centimeter long, alternatively with a particle size from 2 to 50 nanometers. Nanoparticles have very high surface-to-volume ratios. The nanoparticles may be crystalline or amorphous. A single type of nanoparticle may be used, or mixtures of different types of nanoparticles may be used. If a mixture of nanoparticles is used they may be homogeneously or non-homogeneously distributed in the composite material or a system or composition containing the composite material.

Non-limiting examples of suitable particle size distributions of nanoparticles are those within the range of 2 nm to less than 750 nm, alternatively from 2 nm to less than 200 nm, and alternatively from 2 nm to less than 150 nm. It should also be understood that certain particle size distributions may be useful to provide certain benefits, and other ranges of particle size distributions may be useful to provide other benefits (for instance, color enhancement requires a different particle size range than the other properties). The average particle size of a batch of nanoparticles may differ from the particle size distribution of those nanoparticles. For example, a layered synthetic silicate can have an average particle size of about 25 nanometers while its particle size distribution can generally vary between 10 nm to 40 nm. It should be understood that the particle size distributions described herein are for nanoparticles when they are dispersed in an aqueous medium and the average particle size is based on the mean of the particle size distribution.

The nanoparticles used in the invention are exfoliated. In particular, a starting material is exfoliated or disbursed to form the nanoparticles. Such starting material may have an average size of up to 50 microns (50,000 nanometers). The nanoparticle may comprise for example natural or synthetic nanoclays, including those made from amorphous or structured clays.

In one embodiment, the exfoliated nanoparticle is a nanoclay. In a further embodiment, the nanoparticle is a swellable nanoclay or adduct thereof. A swellable nanoclay has weakly bound ions in interlayer positions that may be hydrated or may absorb organic solvents. These swellable nanoclays generally possess a low cationic or anionic charge, i.e. less than 0.9 units of charge per unit cell.

As used herein, "adducts" means oil swellable nanoclays, i.e. those that swell in organic, non-aqueous solvents such as polar and nonpolar solvents. They may be prepared by reacting a water swellable nanoclay with an organic material that binds to the swellable nanoclay. Examples of such binding organic materials include, but are not limited to, a quaternary ammonium compound having the structure:

$$R_1R_2R_3R_4N+ \ X-$$

wherein
$R_1$, $R_2$, $R_3$ and $R_4$ are each independently selected from H, a $C_1$ to $C_{22}$ alkyl, a $C_1$ to $C_{22}$ alkenyl, and a $C_1$ to $C_{22}$ aralkyl, provided that at least one of the R groups is such an alkyl, alkenyl or aralkyl; and
X is the water swellable nanoclay.

The swellable nanoclay may be amorphous or structured, i.e., including sheets or layers, wherein a combination of such layers is referred to as a lattice structure. Examples of suitable nanoclays having lattice structures include the pyrophillite (dioctahedral) type, the talc (trioctahedral) type, or mixtures thereof. Classes of suitable structured swellable nanoclays include, but are not limited to the smectite nanoclays, sepiolite nanoclays, zeolite nanoclays, palygorskite nanoclays, or mixtures thereof.

Examples of amorphous swellable nanoclays include allophone and imogolite. In one embodiment, the nanoparticles are made from a starting material such as Nanomer 1.34TCN (available from Nanocor) having a particle size of 10 to 18 microns (10000 - 18000 nanometers). In another embodiment, the nanoparticles are made from PGV (also available from Nanocor) having a particle size of 20 to 25 microns. In another embodiment, exfoliated PGV having a particle size range of 1-3 nanometers is used. In other embodiments, Nanomer 1.34TCN and Nanomer 1.30E having a particle size range of 1-9 nanometers is used.

Boehmite alumina can have an average particle size distribution from 2 to 750 nm.

Layered clay minerals can be used as starting materials for the exfoliated nanoparticles. The layered clay minerals suitable for use in the present invention include those in the geological classes of the smectites, the kaolins, the illites, the chlorites, the attapulgites and the mixed layer clays. Typical examples of specific clays belonging to these classes are the smectices, kaolins, illites, chlorites, attapulgites and mixed layer clays. Smectites, for example, include montmorillonite, bentonite, pyrophyllite, hectorite, saponite, sauconite, nontronite, talc, beidellite, volchonskoite, stevensite, and vermiculite. In one embodiment, montmorillonite nanoclay is preferred. See United States Patent No. 5,869,033 Kaolins include kaolinite, dickite, nacrite, antigorite, anauxite, halloysite, indellite and chrysotile. Illites include bravaisite, muscovite, paragonite, phlogopite and biotite. Chlorites include corrensite, penninite, donbassite, sudoite, pennine and clinochlore. Attapulgites include sepiolite and polygorskyte. Mixed layer clays include allevardite and vermiculitebiotite. Variants and isomorphic substitutions of these layered clay minerals offer unique applications.

Layered clay minerals may be either naturally occurring or synthetic. For example, natural or synthetic hectorites, montmorillonites and bentonites may be used as the starting material for the nanoparticles.

Natural clay minerals typically exist as layered silicate minerals and less frequently as amorphous minerals. A layered silicate mineral has $SiO_4$ tetrahedral sheets arranged into a two-dimensional network structure. A 2:1 type layered silicate mineral has a laminated structure of several to several tens of silicate sheets having a three layered structure in which a magnesium octahedral sheet or an aluminum octahedral sheet is sandwiched between two sheets of silica tetrahedral sheets.

A sheet of an expandable layer silicate has a negative electric charge, and the electric charge is neutralized by the existence of alkali metal cations and/or alkaline earth metal cations. Smectite or expandable mica can be dispersed in water to form a sol with thixotropic properties. Further, a complex variant of the smectite type clay can be formed by the reaction with various cationic organic or inorganic compounds. An example of such an organic complex, an organophilic clay in which a dimethyldioctadecyl ammonium ion (a quaternary ammonium ion) is introduced by cation exchange. This has been industrially produced and used as a gellant of a coating.

Synthetic nanoclays may be employed in the invention. With appropriate process control, the processes for the production of synthetic nanoclays does indeed yield primary particles that are nanoscale. However, the particles are not usually present in the form of discrete particles, but instead predominantly assume the form of agglomerates due to consolidation of the primary particles. Such agglomerates may reach diameters of several thousand nanometers, such that the desired characteristics associated with the nanoscale nature of the particles cannot be achieved. The particles may be deagglomerated, for example, by grinding as described in EP-A 637,616 or by dispersion in a suitable carrier medium, such as water or water/alcohol and mixtures thereof.

Synthetic materials for making suitable nanoclays include layered hydrous silicate, layered hydrous aluminum silicate, fluorosilicate, mica-montmorillonite, hydrotalcite, lithium magnesium silicate and lithium magnesium fluorosilicate. An example of a substituted variant of lithium magnesium silicate is where the hydroxyl group is partially substituted with fluorine. Lithium and magnesium may also be partially substituted by aluminum. Lithium magnesium silicate may be isomorphically substituted by any member selected from the group consisting of magnesium, aluminum, lithium, iron, chromium, zinc and mixtures thereof.

Synthetic hectorite, for example as commercially marketed under the trade name LAPONITE™ by Southern Clay Products, Inc., may be used as a starting material for the nanoparticles. There are many grades or variants and isomorphous substitutions of LAPONITE™ marketed. Examples of commercial hectorites are LAPONITE B™, LAPONITE S™, LAPONITE XLS™, LAPONITE RD™, LAPONITE XLG™, and LAPONITE RDS.

Synthetic hectorites do not contain any fluorine. An isomorphous substitution of the hydroxyl group with fluorine will produce synthetic clays referred to as sodium magnesium lithium fluorosilicates, which may also be used as the starting material. These sodium magnesium lithium fluorosilicates, marketed as LAPONITE™ and LAPONITE S™, may contain fluoride ions of up to 10% by weight. The fluoride ion content useful in the compositions described herein is up to 10 or more percent. LAPONITE B™, a sodium magnesium lithium fluorosilicate, has a flat, circular, plate-like shape, with an average particle size, depending on fluoride ion content, of 25-100 nanometers. For example, in one non-limiting embodiment, LAPONITE B™ having a diameter of 25-40 nmand and thinkness of about 1 nm may be used. Another variant, called LAPONITE S™, contains about 6% of tetrasodium pyrophosphate as an additive.

In one embodiment, Laponite XLS™ is used as the starting material for the nanoparticle, and silver is loaded thereon as the metal. Laponite XLS has tetrahedral silicate layers joined by octahedral magnesium and lithium hydroxyl bridges. This structure allows for exfoliation and modification by either intercalation or adsorption of metal to the nanoclay surface. In the case of intercalation, the metal is inserted between the layers of nanoclay. In the case of surface adsorption, the metal binds to the surface of the nanoclay. Laponite XLS is advantageous because it is synthetically consistent and pure, and exfoliates to form nanoparticles with minimal effort. The surface of the nanoparticle is covered with sodium ions to balance out the negative charge of the many silicate groups.

The aspect ratio of the exfoliated nanoparticles, in some cases, is of interest in forming films comprising the composite material with desired characteristics. The aspect ratio of dispersions can be adequately characterized by TEM (transmission electron microscopy).

The aspect ratio of nanoparticles in one embodiment can be in the range of 100 to 250. In another embodiment, the aspect ratio of the nanoparticles is 200 to 350.

For example, the average aspect ratio of individual particles of LAPONITE B™ is 20-40 and the average aspect ratio of individual particles of LAPONITE RD™ is 10-15. LAPONITE B™ occurs in dispersions as essentially single clay particles or stacks of two clay particles. LAPONITE RD™ occurs essentially as stacks of two or more single clay particles.

In some embodiments, a high aspect ratio may be desirable for film formation. The aspect ratio of exfoliated nanoparticles dispersed in a suitable carrier medium, such as water, is also of interest. The aspect ratio of the nanoparticles in a dispersed medium is lower where several of the particles are aggregated.

In certain embodiments, it may be desirable for at least some individual (non-aggregated) platelet and disc-shaped nanoparticles to have at least one dimension that is greater than or equal to 0.5 nm, and an aspect ratio of greater than or equal to 15. Larger aspect ratios may be more desirable for platelet and disc-shaped nanoparticles than for rod-shaped nanoparticles.

The aspect ratio of rod-shaped nanoparticles can be lower than that of disc-shaped or platelet-shaped nanoparticles while maintaining adequate film-forming properties. In certain non-limiting embodiments, it may be desirable for at least some of the individual rod-shaped nanoparticles to have at least one dimension that is greater than or equal to 0.5 nm, and an aspect ratio of greater than or equal to 3.

The aspect ratio of spheroid-shaped nanoparticles is generally less than or equal to 5. Nanoparticles preferred for the embodiments presented here have aspect ratios of less than or equal to 250. In other non-limiting embodiments, it may be desirable for the nanoparticles to have an aspect ratio of less than 10.

According to the invention, one or more metals are used to functionalize the nanoparticle. In particular, they are loaded onto the exfoliated nanoparticle by one of a variety of methods including intercalation, adsorption, or ion exchange. Advantageously, the metal retains its valuable properties while on the nanoparticle. The term loaded, as used herein, includes complete coverage of the surface of the nanoparticle, or alternatively, only a portion thereof.

The metal is selected from Groups 3 to 12 of the Periodic Table of Elements, aluminum, and magnesium. Preferably, the metal is selected from silver, copper, zinc, manganese, platinum, palladium, gold, calcium, barium, aluminum, iron, and mixtures thereof. In a particularly preferred embodiment, the metal is silver.

The metal may be loaded onto the nanoparticle via intercalation. For example, silver ions, in particular, can be inserted among the various layers of layered nanoclay by positioning in a "hole" to maximize favorable interactions between the positively charged silver ion and the various types of oxygen in the silicate structure. Intercalation is also possible with other metal ions, such as copper, zinc, manganese, etc for the same reasons.

The metal may also be loaded onto the nanoparticle via ion exchange. For example, the surface of Laponite platelets is composed mainly of sodium ions, which exist to balance out the negatively charged oxygen atoms donated by the silicate structure in the layer below. When positively charged metal ions are added to a solution of exfoliated Laponite, a fraction of the surface sodium ions are displaced by the added metal cations.

The metal may also be loaded onto the nanoparticle by adsorption. For example, certain functional groups such as amine, ammonium, and carboxyl groups are strong binders to the face or edge of a platelet of Laponite. Metal ions can be modified by the addition of these ligands so that they are able to bind strongly to the surface of Laponite. The reaction sequence for one example is shown below:

$$2AgNO_3 + 2NaOH \rightarrow Ag_2O + 2NaNO_3 + H_2O$$

$$Ag_2O + 4NH_3 + H_2O \rightarrow 2Ag(NH_3)_2OH$$

The final product, $Ag(NH_3)_2OH$, is contacted with Laponite, whereby the $Ag(NH_3)_2OH$ binds to the face of the Laponite.

In one embodiment of the invention a metal ion is reduced to a metal (0) in the presence of a starting material, which is exfoliated to form a nanoparticle. Reduction and exfoliation may take place in sequence (either step happening first) or simultaneously upon contacting of the metal with the starting material/exfoliated nanoparticle. The metal is thereby loaded onto the surface of the exfoliated nanoparticle.

In one embodiment of the invention, the metal is silver, which is loaded onto the nanoparticle via intercalation using the Tollen's reagent. The Tollen's reagent is a known silver species able to undergo reduction by either an aldehyde or ketone to form silver metal (0):

$$+Ag(NH_3)_2OH + glucose \rightarrow Ag^0$$

Since nanoparticles possess large surface areas, the composite material will also allow for higher concentrations of metals to be included in the overall formulation.

The composite material of the present invention may be applied directly to the affected area of the skin or via a substrate such as a wipe, diaper, sanitary napkin, liner or the like.

The composite material may be incorporated into a variety of systems, materials and compositions, including liquids, solids, gels, coating compositions, cosmetic and pharmaceutical compositions and the like. The composite material may be incorporated into compositions that are applied directly to the skin of an infant or adult. The compositions of the present invention may be in the form of a cream, lotion, gel, foam, oil, ointment, or powder for topical application to the external skin. The compositions of the present invention may be in a form suitable for application to the skin in either a leave-on product or a rinse-off product. A wipe may be used to deliver the composition.

Compositions containing the composite material may, for example, be in the form of emulsions. Emulsion compositions may comprise, based upon the total weight of the composition, from 1 percent to 50 percent, for example, from 5 percent to 20 percent of at least one oil, from 50 percent to 99 percent, for example, from 80 percent to 95 percent of at least one aqueous component, from 0.1 percent to 20 percent, for example, from 1 percent to 5 percent of the composite material. In general, the composition may contain, based upon the total weight of the composition, from 0.1 percent to 20 percent, for example, from 1 percent to 5 percent of at least one emulsifying agent. The emulsion may be in the form of a lotion, a milk, or a cream.

The oil phase may be comprised of any oil. Examples of suitable oils include, but are not limited to, mineral oil, lanolin, vegetable oils, and mixtures thereof.

The water phase may be comprised of an aqueous component including water, glycols such as propylene glycol, glycerin, dipropylene glycol, and mixtures thereof.

Emulsifying agents that are predominantly hydrophilic in nature are typically added to the aqueous phase, while emulsifying agents that are predominantly lipophilic in nature are typically added to the oil phase. Emulsifying agents suitable for use in the present invention may be at least one nonionic, anionic, cationic, or amphoteric surfactant. Combinations of more than one nonionic, anionic, cationic, or amphoteric surfactant may also be useful. The type of surfactants utilized and the hydrophobe/lypophobe balance of the surfactants utilized will depend on the type of oil-in-water composition desired. It is within the expertise of those of ordinary skill in the art to select surfactant combinations that will provide the desired properties.

Nonionic surfactants useful in this invention include ethoxylated fatty alcohols, ethylene oxide/propylene oxide block copolymers, ethoxylated alkylphenols, ethoxylated or non-ethoxylated esters of alkyl glucose and fatty acid, and glycerol esters.

One class of nonionic surfactants useful in the present invention are polyoxyethylene derivatives of polyol esters, wherein the polyoxyethylene derivative of polyol ester (1) is derived from (a) a fatty acid containing from 8 to 22, and preferably from 10 to 14 carbon atoms, and (b) a polyol selected from sorbitol, sorbitan, glucose, alpha-methyl glucoside, polyglucose having an average of 1 to 3 glucose residues per molecule, glycerine, pentaerythritol and mixtures thereof, (2) contains an average of from 10 to 120, and preferably 20 to 80 oxyethylene units; and (3) has an average of 1 to 3 fatty acid residues per mole of polyoxyethylene derivative of polyol ester.

Examples of polyoxyethylene derivatives of polyol esters include, but are not limited to PEG-80 sorbitan laurate and Polysorbate 20. PEG-80 sorbitan laurate, which is a sorbitan monoester of lauric acid ethoxylated with an average of about 80 moles of ethylene oxide, is available commercially from Uniqema Americas of Wilmington, Del. under the tradename, "Atlas G-4280." Polysorbate 20, which is the laurate monoester of a mixture of sorbitol and sorbitol anhydrides condensed with approximately 20 moles of ethylene oxide, is available commercially from Uniqema Americas of Wilmington, Del. under the trade name "Tween 20."

Another class of suitable nonionic surfactants includes long chain alkyl glucosides or polyglucosides, which are the condensation products of (a) a long chain alcohol containing from 6 to 22, and preferably from 8 to 14 carbon atoms, with (b) glucose or a glucose-containing polymer. The alkyl glucosides have 1 to 6 glucose residues per molecule of alkyl glucoside.

As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. Examples of zwitterionic materials include, but are not limited to, alkyl betaines and amidoalkyl betaines. The amphoteric surfactants are disclosed herein without a counter ion. One skilled in the art would readily recognize that under the pH conditions of compositions comprising the composite material of the present invention, the amphoteric surfactants are either electrically neutral by virtue of having balancing positive and negative charges, or they have counter ions such as alkali metal, alkaline earth, or ammonium counter ions.

Commercially available amphoteric surfactants are suitable for use in the present invention and include, but are not limited to amphocarboxylates, alkyl betaines, amidoalkyl betaines, amidoalkyl sultaines, amphophosphates, phosphobetaines, pyrophosphobetaines, carboxyalkyl alkyl polyamines, and mixtures thereof.

Anionic surfactants are useful in the present invention and may be selected from the following classes of surfactants: alkyl sulfates; alkyl ether sulfates; alkyl monoglyceryl ether sulfates; alkyl monoglyceride sulfates; alkyl monoglyceride sulfonates; alkyl sulfonates; alkylaryl sulfonates; alkyl sulfosuccinates; alkyl ether sulfosuccinates; alkyl sulfosuccinamates; alkyl amidosulfosuccinates; alkyl carboxylates; alkyl amidoethercarboxylates; alkyl succinates; fatty acyl sarcosinates; fatty acyl amino acids; fatty acyl taurates; fatty alkyl sulfoacetates; fatty acids; alkyl phosphates; and mixtures thereof.

In one embodiment, the composite material is contained in a solution having a polymeric emulsifier. As used herein, the term "polymeric emulsifier" refers generally to a polymer having both hydrophilic and hydrophobic moieties that is capable of contributing to the formation of a stable emulsion between an oil phase and an aqueous phase. Any of a variety of suitable polymeric emulsifiers may be used according to the present invention. In certain preferred embodiments, it is desirable to use one or more polymeric emulsifiers that tend to provide shelf stability to the composition into which it is added and/or tend to facilitate the deposition of hydrophobic agent onto a substrate. In addition, certain preferred polymeric emulsifiers comprise those compounds that are water-soluble and are capable of forming a phase stable emulsion, preferably stable for at least about 1 week, more preferably at least about a month, of a hydrophobic agent in water. (As used herein a material is defined as "water-soluble", if it is possible to form a clear solution by adding only 0.5% by weight of the material in deionized water that is stable at room temperature (no settling or phase-instability) for 48 hours.) Certain preferred polymeric emulsifiers are salt-sensitive, in that, their solubility in water is reduced, often dramatically, in the presence of electrolytes (such as electrolytes typically present on the surface of skin). A polymeric emulsifier is defined as "salt-sensitive" if it loses its ability to remain phase stable in aqueous solution when sodium chloride has been added. Specifically, a "salt sensitive" polymeric emulsifier will show phase separation and/or a 30% or more change in viscosity (measured using a Brookfield viscometer with an LVT2 spindle at 12 RPM) if 3% sodium chloride is added to a homogenous solution of 1% (active) polymeric emulsifier in deionized water.

The polymers for use as polymeric emulsifiers in the present invention may be of any suitable molecular weight. In certain embodiments of the invention, the polymeric emulsifier has a weight average molecular weight that is preferably greater than 500,000, more preferably greater than 250,000, and even more preferably greater than 100,000.

Polymeric emulsifiers suitable for the present invention may comprise an associative polymer, i.e., a polymer formed from monomers such that individual repeat units are hydrophilic, such as may be formed by addition polymerization of such as acids as acrylic, methacrylic, maleic, itaconic, and the like or combinations to form copolymers thereof.

Notable commercially available polymeric emulsifiers include, but are not limited to, salt sensitive, hydrophobically modified polyacrylic acid commercially under the tradename Pemulen® TR-1 and TR-2 by Noveon, Inc., water-soluble or water-swellable copolymers based on acrylamidoalkyl sulfonic acid and cyclic N-vinylcarboxamides commercially available under the tradename Aristoflex® AVC by Clariant Corporation; water-soluble or water-swellable copolymers based on acrylamidoalkyl sulfonic acid and hydrophobically modified methacrylic acid commercially available under the tradename Aristoflex® HMB by Clariant Corporation; and a homopolymer of acrylamidoalkyl sulfonic acid commercially available under the tradename Granthix APP by Grant Industries, Inc.

Another class of polymeric emulsifier includes hydrophobically-modified, crosslinked, anionic acrylic copolymers, including random polymers, but may also exist in other forms such as block, star, graft, and the like. In one embodiment, the hydrophobically modified, crosslinked, anionic, acrylic copolymer may be synthesized from at least one acidic monomer and at least one hydrophobic ethylenically unsaturated monomer. Examples of suitable acidic monomers include those ethylenically unsaturated acid monomers that may be neutralized by a base. Examples of suitable hydrophobic ethylenically unsaturated monomers include those that contain a hydrophobic chain having a carbon chain length of at least 3 carbon atoms.

Other materials that may be suitable polymeric emulsifiers include ethylene oxide/ propylene oxide block copolymers, sold under the trade name PLURONIC, available from BASF corporation of Parsippany, N.J., modified cellulose polymers such as those modified cellulose polymers described by the trade name KLUCEL, available from Hercules Corporation of Wilmington, DE.

In certain preferred embodiments of the invention, the compositions comprising the composite material include a salt-sensitive polymeric emulsifier selected from a group consisting of a salt sensitive, hydrophobically modified polyacrylic acid such as Pemulen® TR-1 and TR-2 by Noveon, Inc, an acrylamidoalkyl sulfonic acid, cyclic N-vinylcarboxamides; water-soluble or water-swellable copolymers based on acrylamidoalkyl sulfonic acid and cyclic N-vinylcarboxamides commercially available under the tradename Aristoflex® AVC by Clariant Corporation; water-soluble or water-swellable copolymers based on acrylamidoalkyl sulfonic acid and hydrophobically modified methacrylic acid commercially available under the tradename Aristoflex® HMB by Clariant Corporation and a homopolymer of acrylamidoalkyl sulfonic acid commercially available under the tradename Granthix APP by Grant Industries, Inc.; a hydrophobic agent such as an emollient; e.g., mineral oil, petrolatum, or silicone oil; a wetting agent selected from the group consisting of sulfosuccinates and isethionates; and a hydrophilic particle such as an oxide, silicate, or carbonate, especially zinc oxide or titanium oxide. In an especially preferred embodiment, the composition meets the limitations specified above and also is substantially free of monomeric surfactant emulsifiers.

Any suitable amounts of polymeric emulsifier may be used in compositions comprising the composite material of the present invention. In certain preferred embodiments, the compositions comprise at least 0.3 weight percent, for example, between 0.3% and 3%, between 0.3% and 2%, and between 0.3% and 1%. As used herein and throughout the application, all percents represent percent by weight of active based on the total weight of composition, unless otherwise indicated.

In one embodiment, the polymer emulsifier is combined with a hydrophobic agent. Any of a variety of hydrophobic materials that are water-insoluble, liquid at room temperature, and capable of forming a water-resistant barrier film or coating on a substrate when used in a composition of the present invention, are suitable for use herein as a hydrophobic agent. (As used herein, the term "water-insoluble" refers to a material that when added to deionized water to a concentration by weight of 0.5% (with no other additives) at room temperature, cannot be made to form a clear homogeneous mixture for a period of time lasting at least 48 hours.)

Examples of suitable hydrophobic agents include, but are not limited to materials such as mineral oils, petrolatum, vegetable oils (glyceryl esters of fatty acids, triglycerides), waxes and other mixtures of esters, not necessarily esters of glycerol; polyethylene and non-hydrocarbon based oils such as dimethicone, silicone oils, silicone gums, and the like. In certain embodiments, preferred hydrophobic agents include emollients such as mineral oil, silicone oils, pertrolatum, combinations thereof, and the like.

Any suitable amounts of hydrophobic agent may be used in compositions comprising the composite material of the present invention. In certain embodiments, the hydrophobic agent is present in the composition in a concentration that is at least 0.1 weight percent, such as from 0.1% to 50%, preferably from 0.1 % to 40%, and even more preferably from 0.1% to 30%.

In certain preferred embodiments, the polymeric emulsifier and the hydrophobic agent are present in a respective weight ratio from 60:1 to 1:150, preferably from 40:1 to 1:120, and more preferably from 20:1 to 1:100.

Any material suitable for facilitating the wetting of a substrate with a composition comprising the composite material of the present invention and allowing the hydrophobic agent of the composition to tend to remain on the substrate (for example by avoiding re-emulsification of the hydrophobic agent) may be used as a wetting agent of the present invention. Preferably, suitable wetting agents comprise at least one sulfonate group (moiety) and at least one base-neutralizable carboxylic acid group (moiety).

Non-limiting examples of suitable wetting agents include sulfosuccinates, such as, disodium laureth sulfosuccinate, dioctyl sodium sulfosuccinate, sodium methyl 2-sulfolaurate, and the like, isethiones, such as, sodium cocoyl isethionate, and the like, and sulfoacetates, such as, sodium lauryl sulfoacetate, and the like. A variety of such wetting agents are available commercially from various sources including McIntyre Group Limited (disodium laureth sulfosuccinate sold under the tradename MACKANATE EL, dioctyl sodium sulfosuccinate sold under the tradename MACKANATE DOS 70), Stepan Company (sodium methyl 2-sulfolaurate sold under the tradename ALPHA STEP PC 48, sodium lauryl sulfoacetate sold under the tradename LANTHANOL LAL), and Clariant Corporation (sodium cocoyl isethionate sold under the tradename HOSTAPON SCI 85).

Any suitable amount of wetting agent may be used in the compositions In certain cases, the present compositions comprise at least 0.01 weight percent of wetting agent, preferably from 0.01% to 5%, more preferably from 0.01% to 3%, and even more preferably from 0.1% to 2%.

As used herein, the term "hydrophilic particle" refers generally to any particle (substantially spherical, porous, or high aspect ratio) that tends to be solid at room temperature, is generally hydrophilic in nature, and tends to be either insoluble, sparingly soluble, and/or dissolves in water only at a very slow rate. Any suitable hydrophilic particulate may be used according to the present invention. Applicants have recognized that for certain embodiments, the hydrophilic particulate is preferably selected to be dispersible in water and have a particle diameter of from 0.01 $\mu$m to 500 $\mu$m, preferably from 0.1 $\mu$m to 500 about $\mu$m, more preferably from 0.5 $\mu$m to 400 $\mu$m, more preferably from 1.0 $\mu$m to 300 $\mu$m, and more preferably from 10$\mu$m to 250 $\mu$m. In certain embodiments, the hydrophilic particulate is essentially free of one or more coatings comprising one or more hydrophobic moieties (e.g., coatings of fatty acids, fatty esters, hydrophobic surface modification such as from certain organosilanes, and the like) that tend to cancel the hydrophilic nature of the particulate. Nonlimiting examples of hydrophilic particles suitable for use herein include metal oxides such as zinc oxide, titanium dioxide, transition metal oxides or other oxide pigments, various forms of silica, aluminosilicates, carbonates, combinations of two or more thereof, and the like. For certain preferred embodiments, zinc oxide is particularly notable.

Any suitable amount of hydrophilic particle may be used in the compositions comprising the composite material of the present invention. In certain cases, the compositions comprise from 0.01 percent to 50 percent, preferably from 0.1 percent to 45 percent, and more preferably, from 0.1 percent to 40 percent of a hydrophilic particle.

In certain preferred embodiments, the compositions comprising the composite material of the present invention are substantially free of monomeric surfactant emulsifiers. As used herein, the term "substantially free of monomeric surfactant emulsifiers" refers to a composition comprising less than 1.0%, preferably less than 0.5 percent, more preferably less than 0.1 percent, and even more preferably than 0.01 percent, or less than 0.001 percent. By "monomeric surfactant emulsifiers", it is meant any monomeric surfactant other than those falling under the definition of "wetting agent" above. Examples of monomeric surfactant emulsifiers include nonionic monomeric emulsifiers, such as, for example, polyoxyalkynated alcohols (alcohol alkoxylates) including mixed coconut-oil derived, tallow derived, and synthetic straight-chain - primary, random, or secondary; polyoxyalkynated alkylphenols (alkylphenol alkoxylates) including polyoxyehylenated p-nonylphenol, octyl phenol, and the like.

Other examples of monomeric surfactant emulsifiers include all monomeric emulsifiers that are purely cationic such as long chain amines, diamines, and polyamines and their salts; quaternary ammonium compounds (ethoxylated or non-ehtoxylated, polyoxyethylenated long chain amines, and amine oxides, as well as, amphoteric emulsifiers, i.e., those capable of adopting a zwitterionic state (molecule having both cationic and anionic charges). Examples of these include alkylaminopropionic acids, alkyliminopropionic acids, alkylamphoacetates, alkylamphoglycinates, imidazoline carboxylates, phosphorylated imidazolines, alkylbetaines, alkylamidobetaines, certain amine oxides; sulfobetaines, and alkylsultaines, and alkyl amidosultaines.

The compositions are preferably formulated to be oil-in-water emulsions that are shelf-stable in that the emulsion does not lose phase stability or "break" when kept at standard conditions (22 degrees Celsius, 50% relative humidity) for a week or more after it is made.

The compositions may also include one or more optional ingredients nonexclusively including a pearlescent or opacifying agent, a thickening agent, secondary conditioners, humectants, chelating agents, and additives which enhance their appearance, feel and fragrance, such as colorants, fragrances, preservatives, pH adjusting agents, skin conditioners, anti-irritants, antiinflammatories, anti-microbial agents, sensates, anti-puritics, skin protectants, film formers, preservatives, and the like. The pH of the compositions is preferably maintained in the range of 2 to 10, preferably from 4 to 9, and most preferably from 7 to 9.

Commercially available pearlescent or opacifying agents which are capable of suspending water insoluble additives are suitable for use in this invention. The pearlescent or opacifying agent is present in an amount, based upon the total weight of the composition, of from 0 percent to 3 percent, preferably from 0.25 percent to 2.5 percent, and more preferably, from 0.5 percent to 1.5 percent. Examples of suitable pearlescent or opacifying agents include, but are not limited to

a) mono or diesters of (1) fatty acids having from 16 to 22 carbon atoms and (2) either ethylene or propylene glycol;
b) mono or diesters of (1) fatty acids having from 16 to 22 carbon atoms (2) a polyalkylene glycol of the formula:

$$HO\text{-}(JO)_a\text{-}H,$$

wherein
J is an alkylene group having from 2 to 3 carbon atoms;
and a is 2 or 3;
c) fatty alcohols containing from 16 to 22 carbon atoms;
d) fatty esters of the formula: $KCOOCH_2L$, wherein K and L independently contain from 15 to 21 carbon atoms;
e) inorganic solids insoluble in the composition, and mixtures thereof.

The pearlescent or opacifying agent may be introduced to the composition as a pre-formed, stabilized aqueous dispersion, such as that commercially available from Henkel Corporation of Hoboken, N.J. under the tradename, "Euperlan PK-3000." This material is a combination of glycol distearate (the diester of ethylene glycol and stearic acid), Laureth-4 ($CH_3(CH_2)_{10}CH_2(OCH_2CH_2)_4OH$) and cocamidopropyl betaine and preferably is in a weight percent ratio of from 25 to 30: 3 to 15: 20 to 25, respectively.

Commercially available thickening agents that are capable of imparting the appropriate viscosity to the compositions may be suitable for use in this invention. If used, the thickener should be present in the compositions in an amount sufficient to raise the Brookfield viscosity of the composition to a value of between 500 to 10,000 centipoise. Examples of suitable thickening agents nonexclusively include:

a) mono or diesters of 1) polyethylene glycol of formula:

$$HO\text{-}(CH_2CH_2O)_2H,$$

wherein z is an integer from 3 to 200; and 2) fatty acids containing from 16 to 22 carbon atoms;
b) fatty acid esters of ethoxylated polyols;
c) ethoxylated derivatives of mono and diesters of fatty acids and glycerine;
d) hydroxyalkyl cellulose;
e) alkyl cellulose;
f) hydroxyalkyl alkyl cellulose; and mixtures thereof.

Preferred thickeners include polyethylene glycol ester, and more preferably PEG-150 distearate which is available from the Stepan Company of Northfield, Ill. or from Comiel, S.p.A. of Bologna, Italy under the trade name, "PEG 6000 DS."

Commercially available humectants, which are capable of providing moisturization and conditioning properties to the composition, are suitable for use in the present invention. The humectant is present in an amount of from 0 percent to 10 percent, preferably from 0.5 percent to 5 percent, and more preferably from 0.5 percent to 3 percent, based on the overall weight of the composition. Examples of suitable humectants nonexclusively include: 1) water soluble liquid polyols selected from the group comprising glycerine, propylene glycol, hexylene glycol, butylene glycol, dipropylene glycol, and mixtures thereof; 2) polyalkylene glycol of the formula:

$$HO\text{-}(R''O)_b\text{-}H,$$

wherein R'' is an alkylene group having from 2 to 3 carbon atoms and b is an integer of from 2 to 10;

3) polyethylene glycol ether of methyl glucose of formula

$$CH_3\text{-}C_6H_{10}O_5\text{-}(OCH_2CH_2)_c\text{-}OH,$$

wherein c is an integer from 5 to 25;

4) urea; and 5) mixtures thereof, with glycerine being the preferred humectant.

Preservatives may be useful additives to the formulations of this invention. However and advantageously, they may not be required, due to the anti-microbial nature of silver. Suitable preservatives include Quaternium-15, available commercially as "Dowicil 200" from the Dow Chemical Corporation of Midland, Mich., and are present in the composition in an amount, based upon the total weight of the composition, from 0 to 0.2 percent, and preferably from 0.05 percent to 0.10 percent.

The above described composition may be prepared by combining the desired components in a suitable container and mixing them under ambient conditions in any conventional mixing means well known in the art, such as a mechanically stirred propeller, paddle, and the like. Although the order of mixing is not critical, it is preferable to pre-blend certain components, such as the fragrance and the nonionic surfactant before adding such components into the main mixture.

When using a thickener component, it is also preferable to preblend the desired thickener with from 5 percent to 20 percent, based upon the total weight of the composition, of water and preferably at a temperature of from 60° C to 80° C. When processing with a thickener, it is also preferable to reduce the temperature of the overall composition to less than 45° C. before any pre-formed pearlizer is added thereto.

Alternately, the composite material or a composition comprising the composite material may be incorporated into structures or articles of manufacture such as absorbent articles, wound care articles, or other items comprising soft surfaces.

The composition may be applied to the surface(s) by washing, spraying, dipping, painting, wiping, or by other manner in order to deliver a coating, especially a transparent coating that covers at least 0.5% of the surface, or any greater percentage of the surface, including but not limited to: at least 5%, at least 10%, at least 30%, at least 50%, at least 80%, and at least 100% of the surface. Accordingly, the coating may be continuous or discontinuous.

If the coating composition is to be sprayed onto the surface, the viscosity of the coating composition should be such that it will be capable of passing through the nozzle of a spray device. Such viscosities are well known, and are incorporated herein by reference. The composition may be capable of undergoing shear thinning so that it is capable of being sprayed.

Suitable carrier mediums for the compositions containing the composite material include liquids, solids and gases. One suitable carrier medium is water, which can be distilled, deionized, or tap water. Water is valuable due to its low cost, availability, safety, and compatibility. The pH of the liquid, in particular water, may be adjusted through the addition of acid or base. Aqueous carrier mediums are also easy apply to a substrate and then dried. Though aqueous carrier mediums are more common than dry, nonaqueous mediums, the composition can exist as a dry powder, granule or tablet or encapsulated complex form.

Optionally, in addition to or in place of water, the carrier medium can comprise a low molecular weight organic solvent. Preferably, the solvent is highly soluble in water, e.g., ethanol, methanol, propanol, isopropanol, ethylene glycol, acetone, and the like, and mixtures thereof. The solvent can be used at any suitable level. Several non-limiting examples, include a level of up to 50%, or more; from 0.1% to 25%; from 2% to 15%, and from 5% to 10%, by weight of the total composition. Factors to consider when a high level of solvent is used in the composition are odor, flammability, dispersancy of the nanoparticles and environmental impact.

The carrier medium may also comprise a film former, which when dried, forms a continuous film. Examples of film formers are polyvinyl alcohol, polyethylene oxide, polypropylene oxide, acrylic emulsions, hydroxypropylmethyl cellulose.

Adjunct ingredients that may be used in compositions containing the composite material include polymers and copolymers with at least one segment or group, which comprises functionality that serves to anchor the composite material to a substrate. These polymers may also comprise at least one segment or group that serves to provide additional character to the polymer, such as hydrophilic or hydrophobic properties.

Examples of the anchoring segments or groups include: polyamines, quaternized polyamines, amino groups, quaternized amino groups, and corresponding amine oxides; zwitterionic polymers; polycarboxylates; polyethers; polyhydroxylated polymers; polyphosphonates and polyphosphates; and polymeric chelants.

Examples of the hydrophilizing segments or groups include: ethoxylated or alkoxylated polyamines; polyamines; polycarboxylated polyamines; water soluble polyethers; water soluble polyhydroxylated groups or polymers, including saccharides and polysaccharides; water soluble carboxylates and polycarboxylates; water soluble anionic groups such as carboxylates, sulfonates, sulfates, phosphates, phosphonates and polymers thereof; water soluble amines, quaternaries, amine oxides and polymers thereof; water soluble zwitterionic groups and polymers thereof; water soluble amides and polyamides; and water soluble polymers and copolymers of vinylimidazole and vinylpyrrolidone.

Examples of the hydrophobizing segments or groups include: alkyl, alkylene, and aryl groups, and polymeric aliphatic or aromatic hydrocarbons; fluorocarbons and polymers comprising fluorocarbons; silicones; hydrophobic polyethers such as poly(styrene oxide), poly(propylene oxide), poly(butylene oxide), poly(tetramethylene oxide), and poly(dodecyl glycidyl ether); and hydrophobic polyesters such as polycaprolactone and poly(3-hydroxycarboxylic acids).

Examples of hydrophilic surface polymers that may be incorporated into the compositions of the invention include, but are not limited to: ethoxylated or alkoxylated polyamines; polycarboxylated polyamines; polycarboxylates including but not limited to polyacrylate; polyethers; polyhydroxyl materials; polyphosphates and phosphonates.

Examples of hydrophobic surface polymers that may be incorporated into the compositions of the invention include alkylated polyamines include, but are not limited to: polyethyleneimine alkylated with fatty alkylating agents such as dodecyl bromide, octadecyl bromide, oleyl chloride, dodecyl glycidyl ether and benzyl chloride or mixtures thereof; and polyethyleneimine acylated with fatty acylating agents such as methyl dodecanoate and oleoyl chloride; silicones including, but not limited to: polydimethylsiloxane having pendant aminopropyl or aminoethylaminopropyl groups and fluorinated polymers including, but not limited to: polymers including as monomers (meth)acrylate esters of perfluorinated or highly fluorinated alkyl groups.

Non-polymeric surface modifying materials that may be used as adjunct ingredients include fatty amines and quaternized amines including:

ditallowdimethylammonium chloride; octadecyltrimethylammonium bromide; dioleyl amine; and benzyltetradecyldimethylammonium chloride. Silicone-based surfactants,
fatty zwitterionic surfactants and fatty amine oxides may also be incorporated into the composition.

Another class of adjunct ingredients that may be useful are silicone surfactants and/or silicones. They can be used alone and/or alternatively in combination with other surfactants described herein above. Nonlimiting examples of silicone surfactants are the polyalkylene oxide polysiloxanes having a dimethyl polysiloxane hydrophobic moiety and one or more hydrophilic polyalkylene side chains

The compositions can contain other adjunct ingredients, including but not limited to alkalinity sources, antioxidants, anti-static agents, chelating agents, aminocarboxylate chelators, metallic salts, photoactive inorganic metal oxides, odor-controlling materials, perfumes, photoactivators, polymers, preservatives, processing aids, pigments, and pH control agents, solubilizing agents, zeolites, and mixtures thereof. These optional ingredients may be included at any desired level.

Coating compositions comprising the composite material can be used on all types of soft surfaces, including but not limited to woven fibers, nonwoven fibers, and mixtures thereof. The soft surfaces of interest herein may comprise any known type of soft surface, including but not limited to those associated with disposable absorbent articles including but not limited to covers or topsheets, absorbent cores, transfer layers, absorbent inserts, and backsheets including those outer layers made from breathable and nonbreathable films.

Disposable absorbent articles, such as pantiliners, sanitary napkins, interlabial devices, adult incontinent devices, breast pads, shoe insoles, bandages, and diapers typically are made from absorbent, nonwoven materials (including fibers) and are well known in the art. These articles typically have a fluid permeable body-facing side and fluid impermeable garment facing side. Additionally, such articles may include an absorbent core for retaining fluids therebetween. Addition of the composite material to an article of manufacture such as the absorbent core of a disposable, absorbent article may also help control malodor formation and increase absorbency.

The compositions may also contain a variety of active agents known in the art such as skin lightening agents, skin pigmentation darkening agents, anti-acne agents, sebum modulators, shine control agents, anti-microbial agents, anti-fungals, antiinflammatory agents, anti-mycotic agents, anti-parasite agents, external analgesics, sunscreens, photoprotectors, antioxidants, keratolytic agents, detergents, surfactants, moisturizers, nutrients, vitamins, energy enhancers, anti-perspiration agents, astringents, deodorants, hair removers, firming agents, anti-callous agents, and agents for hair, nail, or skin conditioning.

Formulations for topical or mucosal application are well known in the art. Excipients used by those skilled in the art in such formulations may be used with the composite material herein, provided they are compatible.

In one embodiment, the invention also includes a regimen for treating a person who may at least occasionally wear an absorbent article. The person may continually wear absorbent articles, such as an infant or incontinent adult, or wear absorbent articles only occasionally, such as a child undergoing toilet training or requiring nighttime protection, or an adult who may have a variation of stress incontinence and require occasional protection. Such a regimen may include the following steps: a) removing the absorbent article from the person; b) cleaning the skin of the person to expose an affected area having said dermatitis; and c) coating said affected area with a composition containing a composite material comprising (a) an exfoliated nanoparticle having a surface and (b) a metal selected from Groups 3 to 12, aluminum and magnesium, wherein the metal is loaded onto the surface of the nanoparticle.

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

All percentages are by weight unless otherwise stated.

**Examples**

A series of compositions containing composite materials according to the invention, along with several comparative compositions, were prepared and tested for the ability to inhibit the activity of the enzyme lipase.

The enyzmatic assay of lipase was prepared in general accordance with the Sigma EC 3.1.1.3 procedure, as set forth in "Preparation of Enzymatic Assay of Lipase," Sima-Aldrich Chemical Catalogue, pp 605 (2000/2001). The following Reagents were used to prepare the assay:

A: 200 mM Tris HCl Buffer, pH 7.7 @ 37° C
B: Olive Oil Substrate Solution (50/50 olive oil/water emulsion using Gum Arabic as emulsifier)
C: 95% Ethanol stored at 4° C
D: 0.9% (w/v) Thymolphthalein Indicator Solution
E: 50 mM Sodium Hydroxide Solution
F: Lipase Enzyme Solution in Water

Part 1: Preparation of Assay of Enzyme Activity

Test Solution Preparation: After 2.50 mL of water, 1.00 mL of Reagent A, and 3.00 mL of Reagent B were added to a 50 mL Erlenmeyer flask labeled "TEST," the mixture was equilibrated to 37° C. After 1.00 mL of Reagent F was added thereto, the mixture was mixed and incubated at 37° C for 30 minutes. After 20 mL of Reagent C and 4 drops of Reagent D were added thereto, the mixture was titrated with Reagent E to a light blue endpoint.

Blank Solution Preparation: Into a separate 50 mL Erlenmeyer flask labeled "BLANK" was added 2.50 mL of water, 1.00 mL of Reagent A, and 3.00 mL of Reagent B. After incubating the blank mixture at 37° C for 30 minutes, 20 mL of Reagent C, 1.00 mL of Reagent F, and 4 drops of Reagent D were added thereto. The mixture was then titrated with Reagent E to a light blue endpoint.

Part 2: Preparation of Test Solution:

An appropriate amount of composition was weighed into a 50 mL Erlenmeyer flask labeled "Sample." After adding 2.50 mL of water, 1.00 mL of Reagent A, and 3.00 mL of Reagent B thereto, the resulting mixture was equilibrated to 37° C. After adding 1.00 mL of Reagent F to the flask, the mixture was thoroughly mixed and incubated at 37° C for 60 minutes. After 20 mL of Reagent C and 4 drops of Reagent D were added thereto, the mixture was then titrated with Reagent E to a light blue endpoint.

Blank Solution Preparation: The Blank Solution was prepared as set forth above.

Part 3: Calculation of Enzyme Inhibition Activity:

Enzyme Activity is defined by the following equation:

$$(\text{Reagent E})(\text{Molarity of Reagent E})(1000)(\text{conversion})(\text{df})$$

Wherein:

Reagent E = mL of Reagent E consumed with blank correction, i.e., a correction was made for the amount of NaOH (reagent E) that was required to bring the blank solution to a light blue endpoint in the titration.

Conversion = time conversion factor (e.g. "2" for 30 min. and "_1" for 60 min.)

df = dilution factor = 1

The enzyme activity inhibition for each composition was calculated as the difference between the enzymatic activity of the test solution of Part 1 with the enzymatic activity of each sample solution.

The compositions according to the invention were made as follows. The formation of silver metal from silver ions was completed using $NaBH_4$ as the reducing agent:

$4AgNO_3 + NaBH_4 \rightarrow 4Ag^0$

A solution containing silver-loaded Laponite XLS particles was prepared as follows. 5.0 g of Laponite XLS was added to 1000ml of deonized water. The solution was stirred for 1 hr and labeled solution A (0.5% Laponite XLS solution). To 400ml of solution A, 11mg of $NaBH_4$ was added. This solution was labeled solution B. 21mg of $AgNO_3$ was dissolved in 2ml of deionized water and this was added dropwise to solution B to form a golden yellow solution of 0.002% silver loaded onto Laponite XLS ("Composition 1"). Following the above procedure, 0.004% and 0.005% silver loaded Laponite XLS solutions ("Composition 2" and "Composition 3" respectively) were prepared.

Enzyme (lipase) inhibition was measured for Compositions 1, 2 and 3 as described above, and compared with that of Laponite XLS ("Comparative A"), ZnO at 0.002% (Comparative B), and (Comparative C) $ZnCl_2$ at 0.002%. The results are shown in Tables 1, 2, and 3.

**Table 1**

| Sample | % Enzyme Activity | Standard Deviation | % Enzyme Inhibition (100%-activity) |
|---|---|---|---|
| Comparative A | 92.16% | 2.77% | 7.40% |
| Composition 1 | 72.55% | 2.77% | 27.45% |
| Composition 2 | 51.96% | 4.16% | 48.04% |
| Composition 3 | 43.14% | 5.55% | 56.86% |
| *All Compositions according to the invention v.s. Comparative A significant at P<0.05* | | | |

**Table 2**

| Sample | % Enzyme Activity | Standard Deviation | % Enzyme Inhibition (100%-activity) |
|---|---|---|---|
| Composition 1 | 89.64% | 4.47% | 10.36% |
| Comparative B | 101.38% | 1.81% | -1.38% |
| *Composition 1 according to the invention vs. Comparative B significant at P<0.05* | | | |

**Table 3**

| Sample | % Enzyme Activity | Standard Deviation | % Enzyme Inhibition (100%-activity) |
|---|---|---|---|
| Composition 1 | 89.64% | 4.4% | 10.36% |
| Comparative C | 101.78% | 1.67% | -1.78% |
| *Composition 1 according to the invention vs. Comparative C significant at P<0.05* | | | |

Composition 4 according to the invention was prepared as follows. Composition 1 as made above was formulated into a cream gel by mixing with a base comprising water, Aristoflex® AVC, and mineral oil. Enzyme inhibition of Composition 4 was measured as described above and compared with that of a similar emulsion containing Laponite XLS (without silver) ("Comparative D"). The results arc shown in Table 4.

**Table 4**

| Sample | % Enzyme Activity | Standard Deviation | % Enzyme Inhibition (100%-activity) |
|---|---|---|---|
| Composition 4 | 45.92 | 7.87 | 54.08 |
| Comparative D | 90.13 | 4.46 | 9.87 |
| *Composition 4 vs. Comparative D significant at P< 0.05* | | | |

Two additional formulations (Compositions 5 and 6) according to the invention were also prepared from Composition 1.

Composition 5 was an emulsion comprising Composition 1, steareth-2, steareth-20, cetyl alcohol, mineral oil and water.

Composition 6 was another cream gel comprising Composition 1, water, Aristoflex® AVC, and mineral oil.

The enzyme inhibition of each composition was measured as described above. The results are shown in Table 5.

**Table 5**

| Sample | % Enzyme Activity | Standard Deviation | % Enzyme Inhibition (100%-activity) |
|---|---|---|---|
| Composition 5 | 125.54 | 9.99 | 0 (26% activation) |
| Composition 6 | 36.41 | 0.77 | 63.59 |

Composition 5 did not show activity. This may have been due to the fact that the surfactant system used was uncharged.

**Claims**

1. A composite material comprising: (a) an exfoliated nanoparticle having a surface and (b) a metal selected from Groups 3 to 12, aluminum and magnesium, wherein the metal is loaded onto the surface of the nanoparticle for use in treating enzymatic dermatitis, comprising topically applying to an area of skin affected with enzymatic dermatitis an effective amount of the composite material.

2. The composite material for use in treating enzymatic dermatitis according to claim 1, wherein the metal is selected from the group consisting of silver, copper, zinc, manganese, platinum, palladium, gold, calcium, barium, aluminum, iron, and mixtures thereof, preferably the metal is silver.

3. The composite material for use in treating enzymatic dermatitis according to claim 1, wherein the nanoparticle comprises a nanoclay.

4. The composite material for use in treating enzymatic dermatitis according to claim 1, wherein the nanoparticle comprises exfoliated Laponite.

5. The composite material for use in treating enzymatic dermatitis according to claim 1, wherein said composite material is in the form of a solution.

6. The composite material for use in treating enzymatic dermatitis according to claim 1, wherein said composite material is in the form of a solid.

7. The composite material for use in treating enzymatic dermatitis according to claim 1, wherein said composite material is in the form of a gel.

8. The composite material for use in treating enzymatic dermatitis according to claim 1, further comprising an antibiotic or an antimicrobial.

9. The composite material for use in treating enzymatic dermatitis according to claim 1, further comprising an anti-irritant.

10. The composite material for use in treating enzymatic dermatitis according to claim 1, further comprising an anti-fungal.

**Patentansprüche**

1. Verbundmaterial, umfassend: (a) ein abgeschiefertes Nanoteilchen mit einer Oberfläche und (b) ein Metall, das ausgewählt ist aus den Gruppen 3 bis 12, Aluminium und Magnesium, wobei das Metall auf die Oberfläche des Nanoteilchens geladen ist, zur Verwendung bei der Behandlung enzymatischer Dermatitis, umfassend das topische Aufbringen einer wirksamen Menge des Verbundmaterials auf eine Hautfläche, die von enzymatischer Dermatitis befallen ist.

2. Verbundmaterial zur Verwendung bei der Behandlung enzymatischer Dermatitis nach Anspruch 1, wobei das Metall ausgewählt ist aus der Gruppe, bestehend aus Silber, Kupfer, Zink, Mangan, Platin, Palladium, Gold, Calcium, Barium, Aluminium, Eisen und Mischungen davon, vorzugsweise das Metall Silber ist.

3. Verbundmaterial zur Verwendung bei der Behandlung enzymatischer Dermatitis nach Anspruch 1, wobei das Nanoteilchen einen Nanoton umfasst.

4. Verbundmaterial zur Verwendung bei der Behandlung enzymatischer Dermatitis nach Anspruch 1, wobei das Nanoteilchen abgeschiefertes Laponit umfasst.

5. Verbundmaterial zur Verwendung bei der Behandlung enzymatischer Dermatitis nach Anspruch 1, wobei das Verbundmaterial in der Form einer Lösung vorliegt.

6. Verbundmaterial zur Verwendung bei der Behandlung enzymatischer Dermatitis nach Anspruch 1, wobei das Verbundmaterial in der Form eines Feststoffes vorliegt.

7. Verbundmaterial zur Verwendung bei der Behandlung enzymatischer Dermatitis nach Anspruch 1, wobei das Verbundmaterial in der Form eines Gels vorliegt.

8. Verbundmaterial zur Verwendung bei der Behandlung enzymatischer Dermatitis nach Anspruch 1, das weiter ein Antibiotikum oder ein antimikrobielles Mittel umfasst.

9. Verbundmaterial zur Verwendung bei der Behandlung enzymatischer Dermatitis nach Anspruch 1, das weiter ein reizhemmendes Mittel umfasst.

10. Verbundmaterial zur Verwendung bei der Behandlung enzymatischer Dermatitis nach Anspruch 1, das weiter ein Antipilzmittel umfasst.

**Revendications**

1. Matériau composite comprenant : (a) une nanoparticule exfoliée ayant une surface et (b) un métal choisi dans les groupes 3 à 12, l'aluminium et le magnésium, dans lequel le métal est chargé sur la surface de la nanoparticule pour être utilisé dans le traitement de la dermatite enzymatique, comprenant l'application topique, à une zone de la peau affectée par une dermatite enzymatique, d'une quantité efficace du matériau composite.

2. Matériau composite à utiliser dans le traitement de la dermatite enzymatique selon la revendication 1, dans lequel le métal est choisi dans le groupe constitué d'argent, de cuivre, de zinc, de manganèse, de platine, de palladium, d'or, de calcium, de baryum, d'aluminium, de fer, et de leurs mélanges, de préférence le métal est l'argent.

3. Matériau composite à utiliser dans le traitement de la dermatite enzymatique selon la revendication 1, dans lequel la nanoparticule comprend une nano-argile.

4. Matériau composite à utiliser dans le traitement de la dermatite enzymatique selon la revendication 1, dans lequel la nanoparticule comprend de la Laponite exfoliée.

5. Matériau composite à utiliser dans le traitement de la dermatite enzymatique selon la revendication 1, dans lequel ledit matériau composite est sous la forme d'une solution.

6. Matériau composite à utiliser dans le traitement de la dermatite enzymatique selon la revendication 1, dans lequel ledit matériau composite est sous la forme d'un solide.

7. Matériau composite à utiliser dans le traitement de la dermatite enzymatique selon la revendication 1, dans lequel ledit matériau composite est sous la forme d'un gel.

8. Matériau composite à utiliser dans le traitement de la dermatite enzymatique selon la revendication 1, comprenant en outre un antibiotique ou un agent antimicrobien.

9. Matériau composite à utiliser dans le traitement de la dermatite enzymatique selon la revendication 1, comprenant en outre un anti-irritant.

10. Matériau composite à utiliser dans le traitement de la dermatite enzymatique selon la revendication 1, comprenant en outre un antifongique.

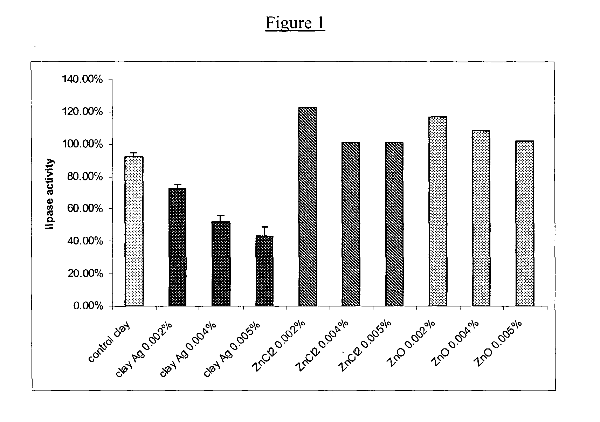

Figure 1

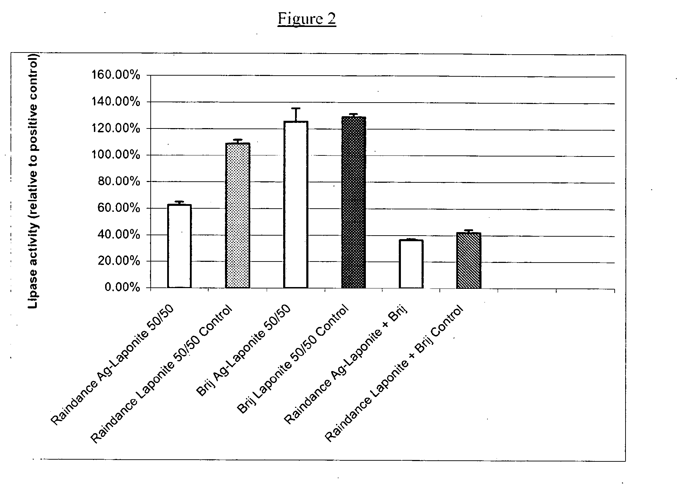

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030104019 A
- US 6492453 B
- US 20030185964 A
- US 6518324 B
- US 20020150678 A
- US 20050129624 A
- US 20050115462 A
- US 5869033 A
- EP 637616 A